# EUROPÄISCHE PATENTANMELDUNG

(11) **EP 0 758 659 A1**
(43) Veröffentlichungstag der Anmeldung: **19.02.1997**
(21) Anmeldenummer: 96112213.2
(22) Anmeldetag: 29.07.1996
(51) Int. Cl.: C08G 18/18, C08G 18/32

(54) **Verfahren zur Herstellung emissionsarmer Polyurethan-Form und -Verbundkörper sowie deren Verwendung**

(30) Priorität: 10.08.1995 DE 19529412
(71) Anmelder: BAYER AG, 51368 Leverkusen (DE)
(72) Erfinder: Haas, Peter, Dr., 42781 Haan (DE); Liman, Ulrich, Dr., 40764 Langenfeld (DE)

(57) **Zusammenfassung**

Beschrieben wird ein Verfahren zur Herstellung von Polyurethan-Formkörper durch Umsetzung von mindestens zwei gegenüber Isocyanaten reaktionsfähige Wasserstoffatome aufweisenden Verbindungen vom Molekulargewicht 400 bis 10.000 sowie gegebenenfalls Kettenverlängerern vom Molekulargewicht 32 ein 399 mit Polyisocyanaten in Gegenwart von gegebenenfalls Treibmitteln, Hilfs- und Zusatzstoffen, bei dem als Aktivatoren bestimmte Carbamate eingesetzt werden. Die nach diesem Verfahren erhaltenen Polyurethane sind besonders emissionsarm, d.h. diffusionsstabil und foggingarm, und eignen sich somit besonders für die Herstellung von Verbundformkörpern aus einem Polyurethan-Kern und mindestens einem weiteren Kunststoffmaterial als Deckschicht. Ebenfalls beschrieben wird die Verwendung dieser diffusionsstabilen und foggingarmen Form- und Verbundkörper, z.B. als Armaturentafeln oder Türverkleidungen im Fahrzeug-, Möbel-, Maschinen- oder Apparatebau.

## Beschreibung

Die Erfindung betrifft ein Verfahren zur Herstellung emissionsarmer diffusionsstabiler Polyurethan-Form- sowie Verbundkörper und deren Verwendung im Fahrzeug-, Möbel-, Maschinen- oder Apparatebau.

Polyurethane, insbesondere Polyurethanschaumstoffe werden in vielen Anwendungsbereichen, z.B. im Möbel- oder Fahrzeugbau als Werkstoffmaterialien eingesetzt.

Neben dem Polyisocyanat und der Polyolkomponente werden bei der Herstellung der Polyurethan-Werkstoffe üblicherweise noch niedermolekulare Verbindungen, z.B. Aktivatoren, zur Abstimmung der Katalyse aus Treib- und Vernetzungsreaktion zugesetzt. Viele dieser bisher als Aktivatoren eingesetzten niedermolekularen Substanzen neigen zu einem unerwünschten Diffusionsverhalten aus dem Polyurethan-Werkstoff.

Eine unerwünschte Diffusion niedermolekularer Substanzen aus dem Polyurethan-Werkstoff kann zum einen dazu führen, daß andere Werkstoffe, welche als Verbundwerkstoffe im direkten Oberflächenkontakt mit Polyurethanen stehen, durch diese ausdiffundierenden Substanzen geschädigt werden. Insbesondere die oft bei der Herstellung von Polyurethan-Werkstoffen eingesetzten Lewis-Basen vom Typ tertiärer Amine können bei einer unerwünschten Diffusion zu einer Schädigung anderer im Verbund mit Polyurethanen eingesetzten Materialien führen.

Bei der Formverschäumung von Polyurethanen ist weiterhin im Fertigungsablauf die häufig längere Formstandzeit im Vergleich zum Spritzguß ein Kostennachteil. Zwar ist durch Verwendung ausreichend hoher Katalysatormengen eine Formstandzeit von 0,5 bis 1,5 Minuten erreichbar, dies führt aber vielfach zu Problemen beim Gebrauch der Artikel, da bisher übliche Katalysatoren, besonders in höherer Konzentration, verstärkt diese unerwünschten Begleiterscheinungen zeigen.

Im Fahrzeugbau kann die unerwünschte Diffusion niedermolekularer Stoffe aus Polyurethan-Werkstoffen weiterhin zu einem sogenannten "Fogging" führen. Unter "Fogging" wird dabei die Kondensation flüchtiger Bestandteile aus der gesamten Kfz-Innenausstattung, z.B. auch aus Armaturentafeln, Mittelkonsolen, Türverkleidungen oder Autohimmeln, an den Innenflächen der Automobil-Glasscheiben bezeichnet. Es handelt sich dabei um einen lichtstreuenden Niederschlag, welcher zu einer Sichtbehinderung, insbesondere bei schlechten Beleuchtungsverhältnissen, führt. So findet heute das Diffusionsverhalten der bei der Polyurethan-Herstellung eingesetzten Additive im Fahrzeugbau besondere Beachtung.

Im Automobilinnenraum eingesetzte Werkstoffe sind zudem einer starken thermischen Beanspruchung ausgesetzt. Besonders stark werden dabei Armaturentafeln thermisch beansprucht, da die heute üblicherweise im Automobilbau eingesetzten stark gekrümmten Frontscheiben wie Sammellinsen wirken und somit den hinter ihnen liegenden Bereich der Armaturentafel besonders stark aufheizen. Armaturentafeln werden heute üblicherweise aus einem geschäumten Polyurethan-Formkörper oft im Verbund mit einer PVC- und/oder ABS-Deckfolie hergestellt. Neben dem bereits erwähnten "Fogging"-Effekt können gegebenenfalls aus dem Polyurethan-Werkstoff ausdiffundierende niedermolekulare Substanzen somit besonders unter diesen extremen Bedingungen zu einer Schädigung dieser im Automobilinnenraum verarbeiteten Werkstoffkombinationen führen. Daher ist es insbesondere im Fahrzeugbau wünschenswert, im Verbund mit anderen Materialien möglichst solche Polyurethan-Werkstoffe einzusetzen, die besonders diffusionsstabil und foggingarm sind.

Ein weiteres Problem der Formverschäumung mit kurzen Reaktionszeiten ist, daß bei der Gasreaktion das entstehende Gas zu schnell und damit mit einem zu hohen Druck im Werkzeug freigesetzt wird. Das führt in vielen Fällen zu Schaumverformungen oder gar Schaumrissen, da die Festigkeit des Polyurethanpolymeres noch nicht ausreicht, den gebildeten Schaumstoff konturstabil zu halten.

Ziel weiterer Entwicklungsarbeiten war es somit, Schaumsysteme bereitzustellen, bei denen die Endwerte der mechanischen Festigkeiten schneller erreicht werden, ohne daß es bei der Katalyse zu Probleme durch Fogging oder Alterung von Deckschichten oder zu Schädigungen der Formteile bei der Entnahme aus der Form infolge verkürzter Entformungszeit kommt.

Aufgabe der vorliegenden Erfindung war es daher, ein Verfahren zur Herstellung emissionsarmer, schnell entformbarer Polyurethan-Formkörper zur Verfügung zu stellen. Eine andere Aufgabe der Erfindung bestand darin, Verbundkörper aus einem Polyurethan-Kern und mindestens einem weiteren Kunststoffmaterial als Deckschicht zur Verfügung zu stellen, die aus einem besonders diffusionsstabilen Polyurethan-Kern bestehen und damit besonders geeignet für den Einsatz im Fahrzeugbau sind.

Überraschend wurde nun gefunden, daß sich besonders emissionsarme und foggingarme Polyurethane mit geringer Formstandzeit herstellen lassen, wenn spezielle niedermolekulare Carbamate als Aktivatoren bei der Herstellung der Polyurethane eingesetzt werden.

Gegenstand der Erfindung ist daher ein Verfahren zur Herstellung von Polyurethanen durch Umsetzung von
a) mindestens 2 gegenüber Isocyanaten reaktionsfähige Wasserstoffatome aufweisenden Verbindungen vom Molekulargewicht 400 bis 10.000 sowie gegebenenfalls Kettenverlängerern vom Molekulargewicht 32 bis 399 mit
b) Polyisocyanaten in Gegenwart von
c) gegebenenfalls Treibmitteln, Hilfs- und Zusatzstoffen
bei dem als Aktivatoren Carbamate der allgemeinen Formel (I) 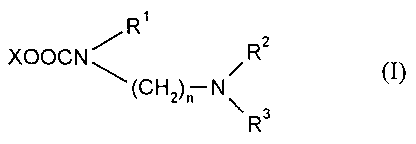 worin
- X: Wasserstoff oder ein Element der 1. Hauptgruppe des Periodensystems,
- n: eine ganze Zahl zwischen 1 und 12,
- R¹: Wasserstoff, ein Alkylrest mit 1 bis 6 Kohlenstoffatomen oder ein N,N-Dimethylaminoalkylrest der Formel -(CH₂)ₘN(CH₃)₂, wobei m eine ganze Zahl zwischen 1 und 6 ist,
- R², R³: unterschiedliche oder identische Alkylreste mit 1 bis 6 Kohlenstoffatomen bedeuten,
oder Carbamate der allgemeinen Formel (II) 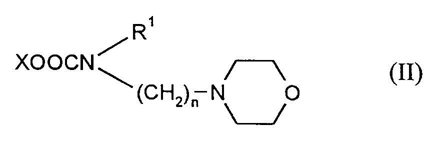 worin
- X, n, R¹: die angegebene Bedeutung besitzen,
oder Carbamate der allgemeinen Formel (III) 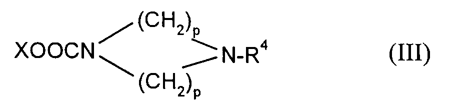 worin
- X: die angegebene Bedeutung und
- p: eine ganze Zahl zwischen 2 und 4,
- R⁴: ein Alkylrest mit 1 bis 2 Kohlenstoffatomen bedeuten,
oder Carbamate der allgemeinen Formel (IV) 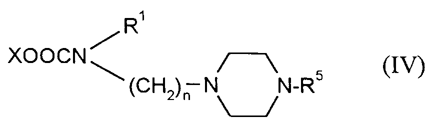 worin
- X, R¹, n: die angegebene Bedeutung besitzen und
- R⁵: ein Alkylrest mit 1 bis 4 Kohlenstoffatomen oder ein Carboxylrest der Formel -(CH₂)_{q}NR¹COOX, wobei q eine ganze Zahl zwischen 1 und 6 ist und R¹ und X die angegebene Bedeutung besitzen, bedeutet,
zugesetzt werden.

Die erfindungsgemäß eingesetzten Aktivatoren zeigen trotz ihrer geringen Größe keinerlei Emissionen, d.h. Wanderungs- und Fogging-Tendenz. Dies zeigt sich z.B. in einem geringen Schädigungsverhalten gegenüber empfindlichen Dekormaterialien und Deckwerkstoffen, wie PVC, ABS, Kombinationen aus PVC und ABS, Polyvinylbutyral, Polyvinylacetat sowie Homo- und Copolymerisaten auf der Basis von Chloropren, Isopren, Styrol, Acrylnitril, Dichlorbutadien, Ethylen, Propylen und Vinylchlorid in Form von Folien, Überzügen, Umrandungen oder Textilien. Durch eine überraschend zügige Katalyse erlauben die erfindungsgemäßen Aktivatoren eine geringe Formverweilzeit.

Die im erfindungsgemäßen Verfahren eingesetzten Aktivatoren sind alle Carbamate, die sich lediglich durch unterschiedlich substituierte Aminoreste unterscheiden.

X kann in allen Verbindungen der angegebenen Formel entweder Wasserstoff oder ein Element der 1. Hauptgruppe, vorzugsweise Natrium, Kalium oder Lithium sein.

Bevorzugt werden Carbamate der Formel (I) zugesetzt, worin n eine ganze Zahl zwischen 1 und 3, R¹ Wasserstoff, ein Alkylrest mit 1 bis 2 Kohlenstoffatomen oder ein N,N-Dimethylaminoalkylrest der angegebenen Formel, in der m eine ganze Zahl zwischen 1 und 3 ist, und R² und R³ unterschiedliche oder identische Alkylreste mit 1 bis 2 Kohlenstoffatomen bedeuten.

Weiterhin werden bevorzugt Carbamate der Formel (II) zugesetzt, worin n eine ganze Zahl zwischen 1 und 3 und R¹ Wasserstoff oder ein Alkylrest mit 1 bis 2 Kohlenstoffatomen bedeutet.

Weiterhin sind Carbamate der Formel (III) bevorzugt, worin p 2 bedeutet.

Ebenfalls bevorzugt sind Carbamate der Formel (IV), worin n eine ganze Zahl zwischen 1 und 3, R¹ Wasserstoff oder ein Alkylrest mit 1 bis 2 Kohlenstoffatomen und R⁵ ein Alkylrest mit 1 bis 2 Kohlenstoffatomen oder ein Carboxylrest der angegebenen Formel, in der q eine ganze Zahl zwischen 1 und 3 ist, bedeuten.

Besonders bevorzugt werden die folgenden Carbamate im erfindungsgemäßen Verfahren eingesetzt:

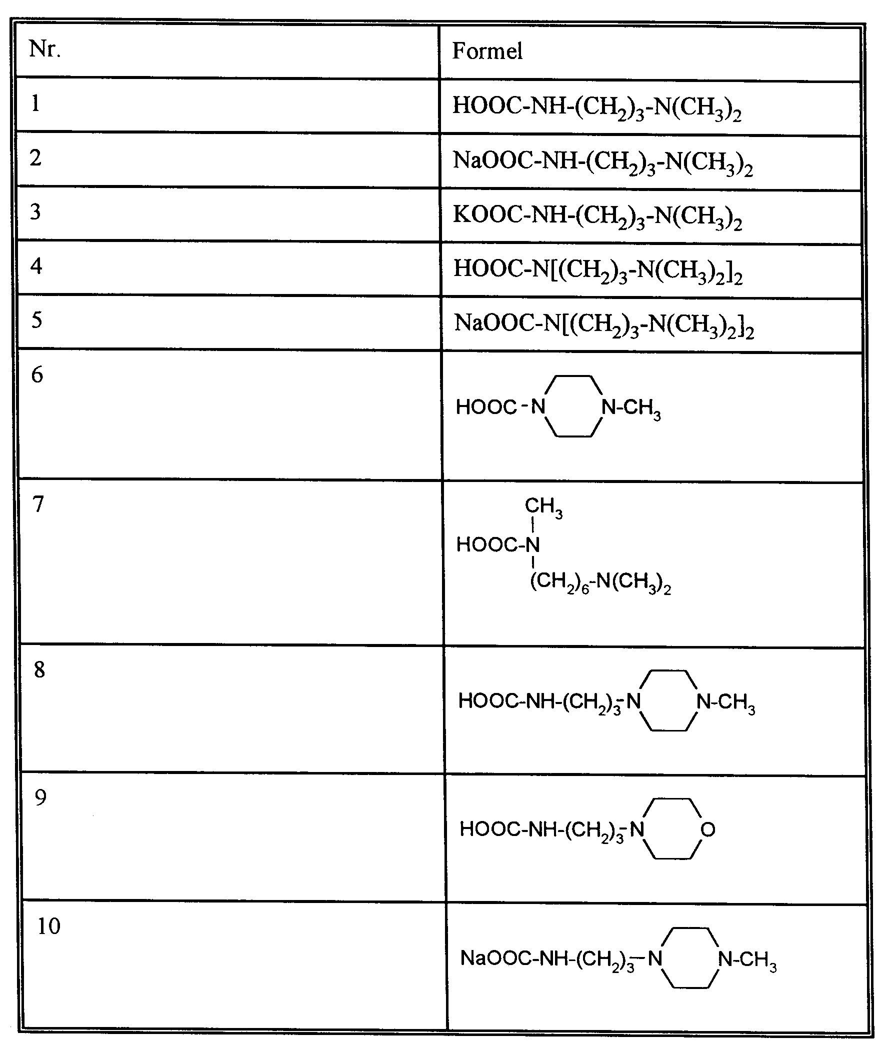

Neben diesen monofunktionellen Carbamaten sind auch solche höherer Funktionalität geeignet, wobei R⁵ in Formel (IV) -(CH₂)_{q}NR¹COOX mit der angegebenen Bedeutung für q R¹ und X sein kann. Ein Beispiel derartiger Carbamate, in dem R¹ und X Wasserstoff sind, ist z.B. die folgende Verbindung:

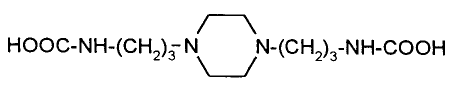

Überraschend bei den sauren Carbamaten Nr. 1, 4, 6 und 7 bis 9, die in Gegenwart von Isocyanaten bekanntlich instabil sind und die Amine zurückbilden können, zeigen diese ein gänzlich anderes Verhalten als es bei den zugrundeliegenden Aminen beobachtet wird, welche selbst völlig ungeeignet sind.

Auch wird das Fließverhalten, das durch einige solcher Aktivatortypen oft in Richtung eines nicht mehr fließfähigen stehenden Schaumes oder sogenannten Froth gedrängt wird, überhaupt nicht beeinflußt. Es tritt im Gegenteil bei der Verwendung der erfindungsgemäßen Aktivatoren in vorteilhafter Weise ein länger niederviskoses und fließfähig bleibendes Reaktionsgemisch auf, das jedoch trotzdem zu schnellem Abbinden neigt.

Die Aktivatoren werden im erfindungsgemäßen Verfahren in einer Menge von 0,01 bis 10 Gew.-%, vorzugsweise 0,1 bis 3,0 Gew.-%, bezogen auf 100 Teile der Polyolkomponente, eingesetzt.

Die Verfahrensdurchführung des erfindungsgemäßen Verfahrens erfolgt in der an sich für Polyurethane bekannten Weise, deren Härtebereich sich von weichen bis hin zu harten Schaumstofftypen erstrecken kann. Erfindungsgemäß können geschäumte oder massive Polyurethane, bevorzugt geschäumte Polyurethan-Formteile hergestellt werden.

Im erfindungsgemäßen Verfahren können alle an sich bekannten Hilfs- und Zusatzstoffe wie z.B. Trennmittel, Treibmittel, Füllstoffe und Flammschutzmittel eingesetzt werden. Gegebenenfalls können auch noch weitere an sich bekannte Aktivatorsubstanzen zugesetzt werden.

Als Treibmittel bei der Herstellung geschäumter Polyurethane kommen Wasser sowie alle hierfür üblichen organischen Treibmittel in Frage.

So kann das erfindungsgemäße Verfahren zur Herstellung von Polyurethankunststoffen erfolgen durch Umsetzung von
a) organischen Polyisocyanaten mit
b) gegenüber Isocyanaten mindestens 2 reaktive Wasserstoffatome enthaltende Verbindungen vom Molekulargewicht 400 bis 10.000, sowie gegebenenfalls gegenüber Isocyanaten mindestens 2 reaktive Wasserstoffatome enthaltende Kettenverlängerer vom Molekulargewicht 32 bis 399,
c) Hilfs- und Zusatzstoffen sowie gegebenenfalls Wasser und/oder niedrigsiedenden Kohlenwasserstoffen als Treibmittel und
d) Carbamaten der angegebenen Formel als Aktivatoren.

Als Ausgangskomponenten werden verwendet:
a) aliphatische, cycloaliphatische, araliphatische, aromatische und heterocyclische Polyisocyanate, wie sie z.B. von W. Siefgen in Justus Liebigs Annalen der Chemie, 362, Seiten 75 bis 136 beschrieben werden, beispielsweise solche der allgemeinen Formel Q(NCO)ₙ, in der
   - n: 2 bis 5, vorzugsweise 2 bis 3, und
   - Q: einen aliphatischen Kohlenwasserstoffrest mit 2 bis 18, vorzugsweise 6 bis 10 Kohlenstoffatomen, einen cycloaliphatischen Kohlenwasserstoffrest mit 4 bis 15, vorzugsweise 5 bis 10 Kohlenstoffatomen, einen aromatischen Kohlenwasserstoffrest mit 6 bis 15, vorzugsweise 6 bis 13 Kohlenstoffatomen bedeuten.

   Besonders bevorzugt werden in der Regel die technisch leicht zugänglichen Polyisocyanate, z.B. das 2,4- und 2,6-Toluylendiisocyanat, sowie beliebige Gemische dieser Isomeren ("TDI"), Diphenylmethandiisocyanat ("MDI") und Polyphenylpolymethylenpolyisocyanate, wie sie durch Anilin-Formaldehyd-Kondensation und anschließende Phosgenierung hergestellt werden und Carbodiimidgruppen, Urethangruppen, Allophanatgruppen, Isocyanuratgruppen, Harnstoffgruppen oder Biuretgruppen aufweisende Polyisocyanate ("modifizierte Polyisocyanate"), insbesondere solche modifizierten Polyisocyanate, die sich vom 2,4- und/oder 2,6-Toluylendiisocyanat bzw. 4,4'-und/oder 2,4'-Diphenylmethandiisocyanat ableiten;
b) als Ausgangskomponenten Verbindungen mit mindestens 2 gegenüber Isocyanaten reaktionsfähigen Wasserstoffatomen mit einem Molekulargewicht von 400 bis 10 000 g/mol, vorzugsweise Polyetherpolyole, d.h. an sich bekannte Additionsprodukte von cyclischen Ethern wie Ethylenoxid, Propylenoxid, Styroloxid, Butylenoxid an Starterverbindungen wie z.B. Polyhydroxyverbindungen wie Alkylenglykole, Glycerin, Trimethylolpropan, Pentaerythrit, Sorbit, Amine wie Ethylendiamin oder Toluylendiamine sowie die Starterverbindungen selbst; Die kettenverlängernden Verbindungen weisen in der Regel 2 bis 8, vorzugsweise 2 bis 4, gegenüber Isocyanaten reaktionsfähige Wasserstoffatome auf und werden in der DE-OS 2 832 253, Seiten 19 bis 20, beschrieben.
c) gegebenenfalls werden als Treibmittel Wasser und/oder Fluoralkane und/oder niedrigsiedende Kohlenwasserstoffe mitverwendet, z.B. niedrigsiedende Alkane wie Pentan, Cycloalkane wie Cyclopentan, ferner Alkene sowie unter Druck in die Reaktionsmischung eingebrachte Gase wie Kohlendioxid;
   gegebenenfalls werden Hilfs- und Zusatzmittel mitverwendet wie oberflächenaktive Zusatzstoffe wie Emulgatoren und Schaumstabilisatoren, Reaktionsverzögerer, Zellregler der an sich bekannten Art wie Paraffine, Fettalkohole oder Dimethylpolysiloxane sowie Pigmente oder Farbstoffe und Flammschutzmittel der an sich bekannten Art, ferner Stabilisatoren gegen Alterungs- und Witterungseinflüsse, Weichmacher und fungistatisch und bakteriostatisch wirkende Substanzen.

Beispiele von gegebenenfalls erfindungsgemäß mitzuverwendenden oberflächenaktiven Zusatzstoffen und Schaumstabilisatoren sowie Reaktionsverzögerern, Stabilisatoren, flammhemmenden Substanzen, Weichmachern, Farbstoffen sowie fungistatisch und bakteriostatisch wirksamen Substanzen sowie Einzelheiten über Verwendungs- und Wirkungsweise dieser Zusatzmittel sind im Kunststoff-Handbuch, Band VII, herausgegeben von G. Oertel, Carl Hanser Verlag, München, 1993, z.B. auf den Seiten 104 bis 127 beschrieben.

Die Carbamate als Aktivatoren können der Polyolkomponente b) zugemischt oder separat der Reaktionsmischung unter Rühren zugesetzt werden.

Ein weiterer Gegenstand der Erfindung sind Verbundkörper aus einem Polyurethan-Kern und mindestens einem weiteren Kunststoffmaterial, wobei der Polyurethan-Kern gemäß dem vorstehend beschriebenen Verfahren unter erfindungsgemäßem Einsatz der genannten Carbamate als Aktivatoren hergestellt wurde. Die erfindungsgemäßen Verbundkörper zeichnen sich dadurch aus, daß aufgrund der erfindungsgemäß hergestellten diffusionsarmen Polyurethan-Kerne praktisch keine Schädigung der Deckschichten durch eventuell aus dem Polyurethan ausdiffundierten niedermolekularen Verbindungen feststellbar sind. Diese Verbundkörper sind somit auch thermisch sehr belastbar. Bevorzugt enthalten die erfindungsgemäßen Verbundkörper Deckschichten aus PVC, ABS, Mischungen von PVC und ABS, Polyvinylacetat, Polyvinylbutyral, Co- und Homopolymerisate auf der Basis von Vinylchlorid, Vinylidenchlorid, Styrol, Butadien, Isopren, Chloropren, Dichlorbutadien, Ethylen, Propylen, Acrylnitril in Form von Folien, Überzügen, Umrandungen und Textilien.

Die erfindungsgemäßen Verbundkörper enthalten den Polyurethan-Kern zumeist in Form einer Hinterschäumung. Beispiele für derartige Verbundkörper sind im Fahrzeugbau eingesetzte Formkörper wie Armaturentafeln, Armlehnen, Konsolen, Nackenstützen, Autohimmel, PKW-Abdeckungen, Automobilsitze und Türverkleidungen.

Ein weiterer Gegenstand der Erfindung ist ein Verfahren zur Herstellung dieser in Sandwich-Bauweise vorliegenden Verbundkörper. Das erfindungsgemäße Verfahren zur Herstellung der Verbundkörper kann dabei sowohl als Depot- oder Hüllbauverfahren ausgestaltet sein. Sowohl die Depotbauweise als auch die Hüllbauweise sind an sich bekannt. Im Depotverfahren (Füllbauweise) werden zwei Halb schalen (z.B. Deckschichten aus faserverstärkten Kunststoffen) vorgefertigt, in ein Werkzeug eingelegt und der Hohlraum zwischen den Schalen mit einem erfindungsgemäßen PUR-Schaum ausgeschäumt. In der Hüllbauweise wird ein Kern aus erfindungsgemäßen PUR-Schaum in einem Werkzeug vorgelegt und dann mit einem geeigneten Hüllmaterial, z.B. mit faserverstärkten Epoxyharzen oder ungesättigten Polyesterharzen, umhüllt. Bei der Herstellung der erfindungsgemäßen Sandwich-Formkörper ist die Hüllbauweise bevorzugt.

Ein weiterer Gegenstand der Erfindung ist die Verwendung der vorstehend beschriebenen Polyurethan-Form- bzw -Verbundkörper im Fahrzeug-, Möbel-, Maschinen- und Apparatebau. Vorzugsweise werden erfindungsgemäße Polyurethan-Verbundkörper mit Sandwichstruktur im Fahrzeugbau, insbesondere im Kfz-Innenraum verwendet.

Die erfindungsgemäßen Polyurethan-Form- und -Verbundkörper sind sehr emissionsarm, d.h. diffusionsstabil und ausgesprochen foggingarm, da praktisch keine niedermolekularen Substanzen aus der erfindungsgemäß hergestellten Polyurethan-Hinterschäumung ausdiffundieren und somit keine eventuellen Schädigungen der darüber- oder darunterliegenden Deckschichten bzw. keine unerwünschten Niederschläge auf den Innenflächen der PKW-Scheiben selbst bei starker thermischer Bedeutung feststellbar sind.

Die nachfolgenden Beispiele sollen die Erfindung näher erläutern, ohne sie jedoch in ihrem Umfang zu begrenzen.

### Beispiele

### A) Herstellung der Carbamate

### Beispiel 1

HOOC-NH(CH₂)₃-N(CH₃)₂

Aus 102 g (1 mol) Dimethylaminopropylamin und 44 g (1 mol) Kohlendioxid in 146 g Wasser. Für die 50 %ige Lösung ergab sich:
Aminzahl: 390, berechnet: 384
Säurezahl: 200, berechnet: 192

### Beispiel 2

NaOOC-NH-(CH₂)₃-N(CH₃)₂

Aus 102 g (1 mol) Dimethylaminopropylamin und 44 g (1 mol) Kohlendioxid; das Carbamat wurde durch 40 g (1 mol) NaOH in Gegenwart von 312 g Wasser in das Na-Salz übergeführt. Für die 35 %ige Lösung ergab sich:
Aminzahl: 125, berechnet: 117

### Beispiel 3

KOOC-NH-(CH₂)₃-N(CH₃)₂

Aus 102 g (1 mol) Dimethylaminopropylamin und 44 g (1 mol) Kohlendioxid; das Carbamat wurde durch 56 g (1 mol) KOH in Gegenwart von 277,5 g Wasser in das K-Salz übergeführt. Für die 40 %ige Lösung ergab sich:
Aminzahl: 130, berechnet: 121

### Beispiel 4

HOOC-N[(CH₂)₃-N(CH₃)₂]₂

Aus 187 g (1 mol) Bis-(dimethylaminopropyl)-amin und 44 g (1 mol) Kohlendioxid in 231 g Wasser. Für die 50 %ige Lösung des Carbamats ergab sich:
Aminzahl: 340, berechnet: 364
Säurezahl: 138, berechnet: 122

### Beispiel 5

NaOOC-N[(CH₂)₃-N(CH₃)₂]₂

Aus 187 g (1 mol) Bis-(dimethylaminopropyl)-amin und 44 g (1 mol) Kohlendioxid; das Carbamat wurde durch 40 g (1 mol) NaOH in Gegenwart von 261 g Wasser in das Na-Salz übergeführt. Für die 40 %ige Lösung ergab sich:
Aminzahl: 188, berechnet: 177

### Beispiel 6

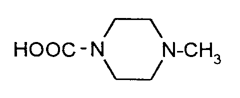

Aus 100 g (1 mol) N-Methylpiperazin und 44 g (1 mol) Kohlendioxid in 144 g Wasser. Für die 50 %ige Lösung ergab sich:
Aminzahl: 410, berechnet: 390
Säurezahl: 180, berechnet: 194

### Beispiel 7

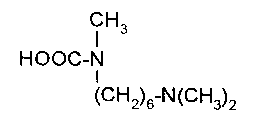

Aus 158 g (1 mol) N,N',N''-Trimethylhexamethylendiamin und 44 g (1 mol) Kohlendioxid in 202 g Wasser. Für die 50 %ige Lösung ergab sich:
Aminzahl: 280, berechnet: 277
Säurezahl: 143, berechnet: 139

### Beispiel 8

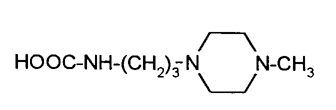

Aus 157 g (1 mol) 3-Aminopropylmethylpiperazin und 44 g (1 mol) Kohlendioxid in 201 g Wasser. Für die 50 %ige Lösung ergab sich:
Aminzahl: 410, berechnet: 418
Säurezahl: 145, berechnet: 139

### B) Erfindungsgemäße Herstellung von Polyurethanen

### Beschreibung der Rohstoffe

Polyetherpolyol 1:
   Polyetherpolyol der OH-Zahl 28 mit einem Gehalt von 17 % Ethylenoxid und 83 % Propylenoxid sowie Trimethylolpropan als Starter mit einem Gehalt von primären OH-Gruppen von mindestens 80 %.
Polyetherpolyol 2:
   Mit Styrol und Acrylnitril (40:60) und einem Füllgrad von 20 Gew.-% gepfropftes Polyetherpolyol der OH-Zahl 28 m it einem Gehalt von 17 % Ethylenoxid und 83 % Propylenoxid sowie Trimethylolpropan als Starter und einem Gehalt an primären OH-Gruppen von mindestens 80 %.
Polyisocyanate:
   Desmodur 44V20 der Bayer AG mit einem NCO-Gehalt von 31,0 %.

Die Herstellung der Polyurethan-Formkörper erfolgte nach einem Verfahren sowie einer Prüftechnik gemäß J. Cell. Plast. Vol. 24, S. 284-298, (1988).

### Beurteilung der erfindungsgemäß hergestellten Formkörper

Als Kriterium für die Formteilentnahme ist die vollständige Bildung des Polymergefüges anzusehen, welche mit der Härte der Polymermatrix korreliert. Diese Härte kann beispielsweise über die Eindrückhärte im Verlauf der Reaktionszeit in Relation zu ihrem Endwert gemessen werden. Ein Formteil wie eine Armaturentafel kann erfahrungsgemäß dann entnommen und weiterverarbeitet werden, wenn ca. 90 % der Endhärte erreicht sind. Hier werden dann keine Verarbeitungsprobleme, wie z.B. Eindruckstellen beim Handling mehr sichtbar.

### Prüfkriterien

### 1. Aushärtungsverhalten

Das Rohstoffgemisch wurde in Handvermischung verarbeitet und in ein auf 40°C beheiztes Metallwerkzeug der Größe 200 x 200 x 40 mm eingebracht. Aus diesen Prüfkörpern wurde anschließend die Abnahme der Eindrückhärte in mm bzw. dieser prozentuale Wert bezüglich des Wertes der Endhärte bei entsprechender Katalyse erfaßt. Hierzu wurde ein Gewicht von 1100 g auf einer Scheibe von 8 mm auf die Schaumstoffe aufgebracht und nach 30 sec die jeweilige Eindrücktiefe als Maß für den Reaktionsfortgang bestimmt. Die Meßzeiten betrugen 3, 5, 10, 20, 30 und 45 Min (vgl. Tabelle 2)

### 2. Alterungsverhalten

Das Alterungsverhalten des Schaumes gegenüber einer angeschäumten Deckschicht der Größe 100 x 100 mm und einer Dicke von 0,8 mm wurde mit
- Folientyp A:: PVC-Haut CZ3 der Fa, LVM, Tessenderlo, Belgien sowie
- Folientyp B:: PVC-/ABS-Haut, Typ LK 55 der Fa. Benecke, Hannover, Deutschland
als Deckschicht ermittelt (vgl. Tabelle 3).

Thermische Belastung im Umlufttrockenofen: 500 Std. bei 120°C.

Hierzu wurden die Folien in einem auf 40° beheizten Metallwerkzeug der Größe 200 x 200 x 40 mm in einer Form mit entsprechender Aussparung für die Folien hinterschäumt und anschließend der Thermobelastung unterzogen.

### 3. Fogging-Verhalten

Zur Beurteilung der Foggingeigenschaften von Werkstoffen der Kfz-Innenausstattung dient die DIN 75 201. Sie beschreibt zwei Methoden:

### Methode A - reflektometrische Methode, Restglanzmessung.

Ein Materialprüfkörper der Abmessungen 80 mm Durchmesser und 10 mm Dicke wird in einem thermostatisierten Gefäß (100°C), welches mit einer besonders gereinigten und ebenfalls thermostatisierten Glasplatte (20°C) abgedichtet ist, 3 Stunden gelagert.

In dem Temperaturintervall 20 bis 100°C verdampf- und kondensierbare niedermolekulare Bestandteile schlagen sich auf der gekühlten Glasplatte nieder und vermindern den Reflexionsgrad im Vergleich zu einer Nullprobe.

### Methode B - gravimetrische Methode

Ein Prüfkörper gleicher Abmessungen wird 16 Stunden in der unter A beschriebenen Apparatur gelagert. Als Kondensationsfläche wird eine gekühlte Alu-Folie (20°C) verwendet, auf der mittels Differenzwägung die kondensierte Masse abzüglich des absorbierten Wassers bestimmt wird.

Das Fogging-Verhalten wurde nach Methode B bestimmt. Die Menge des Niederschlags wurde in mg bestimmt (vgl. Tabelle 4).

Als Vergleichs zum Stand der Technik diente ein mittels Dimethylaminopropylamin als Aktivator unter ansonsten identischen Bedingungen hergestellter Schaum.

### Beispiel 9 (Schaumstoff 1 bis 3)

**Tabelle 1**

| Rezeptur | 1 | 2 | 3 |
|---|---|---|---|
| Polyol 1 | 79 | 79 | 79 |
| Polyol 2 | 17 | 17 | 17 |
| erfindungsgemäßer Aktivator | | | |
| Beispiel 1 | 2,84 | - | - |
| Beispiel 3 | - | 4,11 | - |
| Vergleich | | | |
| Dimethylaminopropylamin | - | - | 1,0 |
| Wasser | 1,08 | 0,24 | 2,5 |
| Schwarzpaste N | 0,5 | 0,5 | 0,5 |
| Kennzahl | 100 | 100 | 100 |
| Isocyanat | 44,7 | 44,7 | 44,7 |
| Rohdichte (kg/m³) | 64 | 68 | 68 |
| Startzeit (sec) | 8 | 10 | 10 |

**Tabelle 2**

| Aushärteverhalten | | | |
|---|---|---|---|
| Schaumstoff gemäß Tabelle 1 Nr. | 1 | 2 | 3 |
| Härte bzw. Eindrücktiefe in % vom Endwert nach: | | | |
| 3 Minuten | 86 | 84 | 68 |
| 5 Minuten | 90 | 90 | 73 |
| 10 Minuten | 95 | 93 | 79 |
| 20 Minuten | 95 | 95 | 90 |
| 30 Minuten | 96 | 96 | 93 |
| 45 Minuten | 98 | 98 | 95 |
| Eindrücktiefe nach 60 Minuten in mm | 3,3 | 4,0 | 5,0 |

**Tabelle 3**

| Folienschädigung nach Kriterium 2 | | | |
|---|---|---|---|
| Schaumstoff gemäß Tabelle 1, Nr.: in Kombination mit | 1 | 2 | 3 |
| Folie A | 1* | 1* | 5* |
| Folie B | 1* | 1* | 5* |

| | | | |
|---|---|---|---|
| * Beurteilung 1: Völlig unbeeinflußt in Mechanik und Farbe 3: Mittlerer Schädigungsgrad in Mechanik und Farbe 5: Starker Schädigungsgrad in Mechanik und Farbe | | | |

**Tabelle 4**

| Foggingverhalten nach DIN 75 201 | | | |
|---|---|---|---|
| Schaumstoff nach Tabelle 1, Nr.: | 1 | 2 | 3 |
| mg Niederschlag | 0,2 | 0,3 | 1,2 |

Aus den Prüfungen geht hervor, daß die erfindungsgemäßen Aktivatoren
a) gemäß Tabelle 2 ein zügiges Aushärteverhalten bewirken. So zeigen die Schaumstoffe 1 und 2 in Tabelle 2 nach 5 Minuten 90 % der Endhärte. Der als Vergleich zum Stand der Technik mit einem Amin hergestellte Schaumstoff 3 zeigt nach 5 Minuten hingegen und 73 % der Endhärte. Der 90 %-Wert wird erst nach 20 Minuten erreicht. Zudem wird die Endhärte bzw. der Vernetzungsgrad ersichtlich in der geringeren Eindrücktiefe nach 60 Minuten deutlich positiv beeinflußt und für Schaumstoff Beispiel 9/1 um ca. 35 % sowie für Beispiel 9/2 um 20 % gegenüber 9/3 reduziert.
b) gemäß Tabelle 3 eine Schädigung der hinterschäumten Folien A und B nach 500 Stunden bei 120°C nicht erkennen lassen,
c) gemäß Tabelle 4 ein ausgesprochen Fogging-armes Verhalten zeigen.

Eine Übertragung der Rezepturen auf die Verarbeitung in Hochdruckmischaggregaten, wie sie produktionstechnisch üblich sind (z.B. beschrieben im Kunststoff-Handbuch, Band (VII), herausgegeben von G. Oertel, Carl-Hanser-Verlag, München, 1993, Seite 143 ff.), zeigte, daß Formstandzeiten unter 60 Sekunden für die Fertigung einer kompletten Instrumententafel erreicht werden.

Die erfindungsgemäße Katalyse ermöglicht somit kürzeste Formstandzeiten in Kombination mit herausragenden Alterungseigenschaften bezüglich Emissionen wie Diffusion und Fogging.

## Patentansprüche

1. Verfahren zur Herstellung von Polyurethanen durch Umsetzung von
a) mindestens 2 gegenüber Isocyanaten reaktionsfähige Wasserstoffatome aufweisenden Verbindungen vom Molekulargewicht 400 bis 10.000 sowie gegebenenfalls Kettenverlängerern vom Molekulargewicht 32 bis 399 mit
b) Polyisocyanaten in Gegenwart von
c) gegebenenfalls Treibmitteln, Hilfs- und Zusatzstoffen
dadurch gekennzeichnet, daß als Aktivatoren Carbamate der allgemeinen Formel (I) 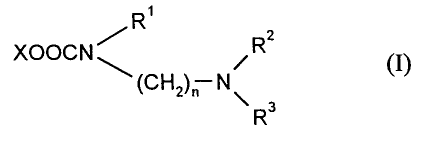 worin
X Wasserstoff oder ein Element der 1. Hauptgruppe des Periodensystems,
n eine ganze Zahl zwischen 1 und 12,
R¹ Wasserstoff, ein Alkylrest mit 1 bis 6 Kohlenstoffatomen oder ein N,N-Dimethylaminoalkylrest der Formel -(CH₂)ₘN(CH₃)₂, wobei m eine ganze Zahl zwischen 1 und 6 ist,
R², R³ unterschiedliche oder identische Alkylreste mit 1 bis 6 Kohlenstoffatomen bedeuten,
oder Carbamate der allgemeinen Formel (II) 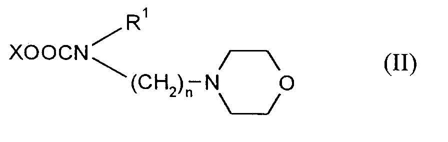 worin
X, n, R¹ die angegebene Bedeutung besitzen,
oder Carbamate der allgemeinen Formel (III) 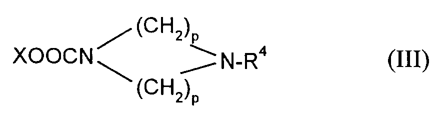 worin
X die angegebene Bedeutung und
p eine ganze Zahl zwischen 2 und 4,
R⁴ ein Alkylrest mit 1 bis 2 Kohlenstoffatomen bedeuten,
oder Carbamate der allgemeinen Formel (IV) 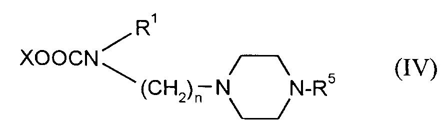 worin
X, R¹, n die angegebene Bedeutung besitzen und
R⁵ ein Alkylrest mit 1 bis 4 Kohlenstoffatomen oder ein Carboxylrest der Formel -(CH₂)_{q}NR¹COOX, wobei q eine ganze Zahl zwischen 1 und 6 ist, und R¹ und X die angegebene Bedeutung besitzen, bedeutet,
zugesetzt werden.

2. Verfahren nach Anspruch 1, dadurch gekennzeichnet, daß Carbamate der Formel (I), worin n eine ganze Zahl zwischen 1 und 3, R¹ Wasserstoff, ein Alkylrest mit 1 bis 2 Kohlenstoffatomen oder ein N,N-Dimethylaminoalkylrest der angegebenen Formel, in der m eine ganze Zahl zwischen 1 und 3 ist, und R² und R³ unterschiedliche oder identische Alkylreste mit 1 bis 2 Kohlenstoffatomen bedeuten, zugesetzt werden.

3. Verfahren nach Anspruch 1, dadurch gekennzeichnet, daß Carbamate der Formel (II), worin n eine ganze Zahl zwischen 1 und 3 und R¹ Wasserstoff oder ein Alkylrest mit 1 bis 2 Kohlenstoffatomen bedeutet, zugesetzt werden.

4. Verfahren nach Anspruch 1, dadurch gekennzeichnet, daß Carbamate der Formel (III), worin p 2 bedeutet, zugesetzt werden.

5. Verfahren nach Anspruch 1, dadurch gekennzeichnet, daß Carbamate der Formel (IV), worin n eine ganze Zahl zwischen 1 und 3, R¹ Wasserstoff oder ein Alkylrest mit 1 bis 2 Kohlenstoffatomen und R⁵ ein Alkylrest mit 1 bis 2 Kohlenstoffatomen oder ein Carboxylrest der angegebenen Formel, in der q eine ganze Zahl zwischen 1 und 3 ist, bedeuten, zugesetzt werden.

6. Verfahren nach einem der vorherigen Ansprüche, dadurch gekennzeichnet, daß die Carbamate in einer Menge von 0,01 bis 10, bevorzugt 0,1 bis 3,0 Gew.-%, bezogen auf 100 Teile der Polyolkomponente, zugesetzt werden.

7. Verfahren nach einen der vorherigen Ansprüche, dadurch gekennzeichnet, daß geschäumte Polyurethan-Formkörper hergestellt werden.

8. Verbundkörper aus einem Polyurethan-Kern und mindestens einem weiteren Kunststoffmaterial als Deckschicht, dadurch gekennzeichnet, daß der Polyurethan-Kern aus gemäß einem der Ansprüche 1 bis 7 erhältlichen Polyurethan besteht.

9. Verbundkörper nach Anspruch 8, dadurch gekennzeichnet, daß die Deckschicht aus PVC, ABS, Polyvinylacetat, Polyvinylbutyral, Co- oder Homopolymerisaten auf der Basis von Vinylchlorid, Vinylidenchlorid, Styrol, Butadien, Isopren, Chloropren, Dichlorbutadien, Polyethylen, Polypropylen, Acrylnitril oder üblichen Mischungen hieraus besteht.

10. Verfahren zur Herstellung eines Verbundkörpers aus einem Polyurethan-Kern und einem weiteren Kunststoffmaterial als Deckschicht, dadurch gekennzeichnet, daß auf einem Kern, bestehend aus einem gemäß einem der Ansprüche 1 bis 7 erhältlichen Polyurethan, eine Deckschicht aus einem anderen Kunststoffmaterial in Sandwichbauweise aufgetragen wird.

11. Verwendung der Verbundkörper gemäß einem der Ansprüche 8 bis 9 im Fahrzeug-, Möbel-, Maschinen- oder Apparatebau.
